(19) Europäisches Patentamt
European Patent Office
Office européen des brevets

(11) **EP 1 790 336 A1**

(12) **EUROPEAN PATENT APPLICATION**
published in accordance with Art. 158(3) EPC

(43) Date of publication:
**30.05.2007   Bulletin 2007/22**

(21) Application number: **05767074.7**

(22) Date of filing: **29.07.2005**

(51) Int Cl.:
*A61K 9/64* (2006.01)      *A61K 9/56* (2006.01)
*A61K 31/19* (2006.01)      *A61K 31/661* (2006.01)
*A61K 31/7008* (2006.01)      *A61P 3/00* (2006.01)
*A23L 1/00* (2006.01)

(86) International application number:
**PCT/JP2005/013916**

(87) International publication number:
**WO 2006/011592 (02.02.2006 Gazette 2006/05)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.07.2004   JP 2004223745**
**27.04.2005   JP 2005129968**

(71) Applicant: **WAKUNAGA PHARMACEUTICAL CO., LTD.**
**Osaka-shi,**
**Osaka 532-0003 (JP)**

(72) Inventors:
• **SHIMOKAWA, Yoshiyuki,**
**Wakunaga Pharma. Co., Ltd.**
**Akitakata-shi, Hiroshima,**
**739-1195 (JP)**

• **SHIRAISHI, Sumihiro,**
**Wakunaga Pharma. Co., Ltd.**
**Akitakata-shi, Hiroshima,**
**739-1195 (JP)**
• **MIHARA, Mika,**
**Wakunaga Pharma. Co., Ltd.**
**Akitakata-shi, Hiroshima,**
**739-1195 (JP)**

(74) Representative: **Haug, Dietmar et al**
**Andrae Flach Haug**
**Balanstrasse 55**
**81541 München (DE)**

(54) **COMPOSITION FOR CAPSULE FILM, CAPSULE FILM, AND CAPSULE MADE WITH THE SAME**

(57)    The present invention provides a composition for capsule coating which can highly suppress the decrease in gelatin-coating solubility over time, and particularly, can also impart a sufficient capsular strength when is applied to a soft capsule, and also provides a capsule coating and a capsule using the same. The composition for capsule coating is **characterized by** containing a gelatin (A) and an inositol phosphate (B) represented by a general formula: $C_6H12\text{-}n \cdot (H_2PO_4)_n$ (wherein n represents an integer from 1 to 6).

EP 1 790 336 A1

**Description**

TECHNICAL FIELD

The present invention relates to a composition for capsule coating containing a gelatin, a capsule coating, and a capsule using the same.

The present invention claims priority on Japanese Patent Application No. 2004-223745 filed on July 30, 2004, and Japanese Patent Application No. 2005-129968 filed on April 27, 2005, the content of which is incorporated herein by reference.

BACKGROUND ART

In the field of food and drugs, gelatin capsules using gelatin for the coating thereof are usually used in terms of safety, rapid solubility inside the body, and the like. However, cross-linking caused by interaction between a gelatin molecule and at least one of filled contents and decomposed matter thereof tends to cause a decrease in the coating solubility of gelatin capsules, which influences the bioavailability of active ingredients. This problem significantly occurs when the filled contents include galenicals, higher unsaturated fatty acids such as DHA, EPA, or the like, unsaturated fatty acid residue-containing oils and fats, minerals, mineral-containing yeasts, vitamin C, and the like, in particular.

In order to solve the above-mentioned problem, (1) the addition of amino acids, citric acids, tartaric acids, or fumaric acids to gelatin coating (Patent Document 1 and Patent Document 2), (2) the addition of pullulan and polypeptide to gelatin coating (Patent Document 3), and the like have been proposed.

Moreover, it is known as a general problem of a soft capsule that the adhesion thickness of a coating joined portion cannot be sufficiently obtained when the capsule is formed, and so the strength of the capsule becomes insufficient. Therefore, leakage of the contents of the capsule, rupture of the capsule, or the like is caused.

[Patent Document 1] Japanese Examined Patent Application, Second Publication No. Sho 57-30088

[Patent Document 2] Japanese Unexamined Patent Application, First Publication No. Sho 59-39834

[Patent Document 3] Japanese Unexamined Patent Application, First Publication No. Hei 05-65222

DISCLOSURE OF THE INVENTION

[Problems to be Solved by the Invention]

However, the above-mentioned prior arts (1) and (2) have not yet resulted in sufficient prevention of the decrease in the coating solubility.

Also, the effects of the improvement in the strength of the soft capsule are not necessarily sufficient.

The present invention has been achieved in view of the above-mentioned circumstances, and has as an object thereof to provide a composition for a capsule coating which can highly suppress the decrease in the coating solubility over time, and can particularly give a sufficient capsular strength in the case of a soft capsule, and provide a capsule coating and a capsule using the same.

[Means for Solving the Problems]

The inventors of the present invention found that the above-mentioned problem can be solved by formulating an inositol phosphate into a capsule coating, and thus arrived at the present invention.

That is, a composition for capsule coating according to the present invention is characterized by containing a gelatin (A) and an inositol phosphate (B) represented by the general formula: $C_6H_{12-n} \cdot (H_2PO_4)_n$ (in the formula, n represents an integer from 1 to 6).

A capsule coating according to the present invention is characterized by containing a gelatin (A) and an inositol phosphate (B) represented by the general formula: $C_6H_{12-n} \cdot (H_2PO_4)_n$ (in the formula, n represents an integer from 1 to 6). In the capsule coating according to the present invention, it is preferable that the component (B) contain an inositol hexaphosphate.

A capsule according to the present invention is characterized by including the capsule coating according to the present invention. The capsule according to the present invention is particularly effective when a filled content thereof contains at least one selected from the group consisting of galenicals, unsaturated fatty acids, unsaturated fatty acid residue-containing oils and fats, minerals, mineral-containing yeasts, and vitamin C. Also, it is preferable that the capsule according to the present invention has a filled content containing at least one selected from the group consisting of phospholipids, amino sugars, and organic acids. Moreover, the capsule according to the present invention is particularly effective in the case of a soft capsule.

The capsule according to the present invention may be a soft capsule.

A production method of the soft capsule is characterized by including a production step of the composition for capsule coating containing at least a gelatin (A) and an inositol phosphate (B) represented by the general formula: $C_6H_{12-n} \cdot (H_2PO_4)_n$ (in the formula, n represents an integer from 1 to 6), and an encapsulation step in which the capsule coating is produced from the composition for capsule coating and a content to be filled in the capsule is interposed between the capsule coating followed by conducting pressure bonding.

Also, the above-mentioned production method may further include a step of preparing the content to be filled containing at least one selected from the group consisting of phospholipids, amino sugars, and organic acids.

[Effects of the Invention]

According to the present invention, a composition for capsule coating which can highly suppress the decrease in gelatin-coating solubility over time, and can particularly impart a sufficient capsular strength in the case of a soft capsule, as well as a capsule coating and a capsule using the same can be provided.

BEST MODE FOR CARRYING OUT THE INVENTION

In the following, the present invention will be explained in detail. "Composition for capsule coating and capsule coating"

The composition for the capsule coating according to the present invention is a composition used for producing a capsule coating, and is characterized by containing at least a gelatin (A) and an inositol phosphate (B) represented by the general formula: $C_6H_{12-n} \cdot (H_2PO_4)_n$ (in the formula, n represents an integer from 1 to 6).

The capsule coating according to the present invention is characterized by containing the gelatin (A) and the inositol phosphate (B) represented by the general formula: $C_6H_{12-n} \cdot (H_2PO_4)_n$ (in the formula, n represents an integer from 1 to 6).

The inventors of the present invention found that addition of the inositol phosphate to the gelatin coating significantly prevents the decrease in coating solubility over time, that is, the decrease caused by, for example, cross-linking generated by interaction between a gelatin molecule and filled contents or decomposed matter thereof, and, in the case of a soft capsule, the addition causes joined portions of coating to be bonded with a sufficient thickness. Therefore, the addition can impart a sufficient capsular strength and prevent leakage of the content from the joined portions of the coating, the rupture of the capsule, or the like.

The gelatin (A), which is a main component, is not particularly limited, and well-known gelatins used for general capsules, such as acid-treated gelatins, alkali-treated gelatins, amphoterically-treated gelatins, chemically-modified gelatins, or the like may be used. These may be used alone or in combination with two or more kinds thereof.

The gelatin is extracted after hydrolyzing a collagen, and, as a hydrolytic agent, the "acid-treated gelatin" uses an acid such as hydrochloric acid, sulfuric acid, or the like, the "alkali-treated gelatin" uses alkalis such as lime or the like, and the "amphoterically-treated gelatin" uses both the acid and the alkali. Moreover, the "chemically-modified gelatin" is one obtained by reacting an amino group of the gelatin with an organic acid such as succinic acid, phthalic acid, or the like. Among them, the acid-treated gelatin and the alkali-treated gelatin are preferably used.

The inositol phosphate (B) is one represented by the above-formula, and examples thereof include inositol monophosphate, inositol diphosphate, inositol triphosphate, inositol tetraphosphate, inositol pentaphosphate, and inositol hexaphosphate (phytic acid), each of which has 1 to 6 phosphate groups inserted therein (n=1 to 6). These may be used alone or in combination with two or more kinds thereof.

Among them, it is preferable to use as the component (B) inositol phosphates having 3 to 6 phosphate groups (n=3 to 6), and particularly phytic acid having 6 phosphate groups (n=6), because effects of preventing the decrease in the solubility and increasing strength are excellently exhibited.

The amount of the inositol phosphate (B) added is not particularly limited. However, when the amount of the component (B) is extremely low, effects obtained by adding the component (effects of preventing the decrease in solubility and increasing the strength) may not be sufficiently exhibited. On the other hand, when the amount is extremely high, particularly in the case of the soft capsule, the decrease in capsular strength or adhesion between capsules may be caused by decreasing the pH of the coating or relatively decreasing the amount of gelatin. Accordingly, it is preferable that the amount of the inositol phosphate (B) contained in the capsule coating be 0.05 to 15% by mass, more preferably 1 to 10% by mass, and even more preferably 2 to 8% by mass, with respect to the amount of the gelatin (A).

In addition to the component (A) and the component (B), various additives generally used for capsule coatings, such as, for example, plasticizers such as glycerin or sorbitol for components other than the component (B), such as amino acids, citric acids, or the like, antiseptics, coloring agents such as dyes or titanium oxide, organic acids, or the like may be contained in the composition for capsule coating and the capsule coating according to the present invention, as needed.

The composition for capsule coating can be produced by mixing and dissolving the components (A) and (B), and further various additives, as needed, in water at room temperature or while heating.

In brief explanation, the capsule coating can be produced by making the composition for capsule coating into a film, forming the film into a predetermined shape, and then drying. Although the content of water contained in the capsule coating after drying is not particularly limited, 5 to 20% by mass, particularly 7 to 15% by mass is preferable. The time for forming the capsule coating and filling the content thereof is determined depending on the type of the capsule. Although the present invention is applicable for both soft capsules and hard capsules, the soft capsules are particularly preferable.

The "soft capsule" is produced by formulating a plasticizer such as glycerin, sorbitol, or the like, encapsulating a content to be filled by a film-like capsule coating which is relatively soft with increased plasticity, and forming it into a predetermined shape at the same time as or after encapsulating the content (encapsulation forming). The "hard capsule" is produced by directly filling a content to be filled into a capsule coating which is manufactured into a predetermined shape with relative hardness in advance, or by slightly conducting shape forming after filling the content.

The present invention is particularly effective in the case of the soft capsule which relatively tends to generate cross-linking caused by interaction between a gelatin molecule and the filled content or decomposed matter thereof.

In the case of the soft capsule, the capsule can be produced by, for example, processing the composition for capsule coating into a film using a rotatory type soft capsule filler, supplying the film into a roll-metal mold from both left and right sides thereof, inserting while pressing a content to be filled into it just before punching it out into a predetermined shape, conducting shape forming, and drying.

In the case of the hard capsule, an immersion method in which a stainless-steel mold-pin is immersed in an immersion fluid prepared by dissolving a gelatin in purified water while stirring and adding inositol phophate to the solution, and the mold-pin is rotated in order to be dried can be used, for example.

The composition for capsule coating and the capsule coating according to the present invention can significantly prevent the decrease in the solubility of the gelatin coating over time by formulating inositol phosphate (B), and, particularly, when the capsule coating is applied to a soft capsule, a sufficient strength can be further given by increasing the thickness of the joined portion of the capsule coating.

The technology of the present invention enables the use of conventional methods without a change in the production of a capsule except that inositol phosphate (B) is added to a conventional coating composition, and therefore, is preferable.

"Capsule"

A capsule according to the present invention is characterized by including the above-mentioned capsule coating of the present invention. Although the capsule may be either a soft capsule or a hard capsule as described above , the soft capsule is particularly preferable.

The form of the filled content is not particularly limited, and it may be a liquid, suspension, paste, powder, granular, or the like. In the case of the granular, coating may be formed using a coating agent.

The capsule of the present invention can be used for various applications such as pharmaceuticals, quasi-drugs, health foods, general foods, cosmetics, or the like, and the constitution of the filled content is arbitrarily determined depending on the application of the capsule.

In the present invention, formulation of inositol phosphate (B) into the capsule coating can significantly inhibit the decrease in the coating solubility over time, and, particularly, when the capsule coating is applied to a soft capsule, the thickness of the joined portion of the capsule coating can be increased, so a sufficient strength can be further given and effects can be exhibited in spite of the constitution of the filled content.

Moreover, the inventors of the present invention have found that when at least one selected from the group consisting of phospholipids, amino sugars, and organic acids is added to the filled content, effects exhibited by the filled content on the capsule coating decrease, so the coating solubility can be further prevented from decreasing over time.

The "phospholipid" is not particularly limited, and examples thereof include phosphatidyl serine, phosphatidylcholine (lecithin), phosphatidylinositol, phosphatidylethanolamine (cephalin), phosphatidylcardiolipin, phosphatidic acid, sphingomyelin, derivatives thereof, and the like. These may be used alone or in combination with two or more kinds thereof.

Among them, phosphatidylethanolamine and derivatives thereof are preferable in view of their excellent effects of preventing the decrease in solubility. Examples of the derivatives of phosphatidylethanolamine include a monoacyl type (lyzo type), alkenyl type, N-methyl type and N, N-dimethyl type in which base portions are methylated, N-acyl type, and the like, of a phosphatidylethanolamine that is a diacyl type.

As phospholipid-containing products containing at least one of the above-mentioned phospholipids, soybean phospholipid (soybean lecithin), yolk phospholipid (yolk lecithin), and the like are commercially available, and these are easily obtained and exhibit excellent effects, and so are preferably used. These may be purified products or crudely purified products, and also may be hydrogenated products or powdered products.

The "amino sugar" is not particularly limited, and saccharides containing an amino group such as glucosamine, N-acetyl glucosamine, galactosamine, N-acetyl galactosamine, N-acetyl neuraminic acid, and the like, or polymers of glucosamine can be used, for example. Specific examples thereof include compounds having a structure in which a hydroxyl group of the saccharide is replaced with an amino group, such as chitosan and the derivatives thereof, polygalactosamine and derivatives thereof. Examples of chitosan and the derivatives thereof include low-molecular-weight chitosan, high-molecular-weight chitosan, chitosan oligosaccharide, and the like, and all those obtained by deacetylating chitins are preferably used. The amino sugars may be used alone or in combination with two or more kinds thereof.

The "organic acid" is not particularly limited, and examples thereof include amino acids such as tryptophan, aspartic acid, glutamic acid, glycine, phenylalanine, arginine, lysine, and the like, citric acid, succinic acid, fumaric acid, tartaric acid, lactic acid, malic acid, inositol phosphate, and the like. These may be used alone or in combination with two or more kinds thereof.

Among them, inositol phosphate and citric acid are preferable in view of their excellent inhibitory effects against the decrease in the solubility. As inositol phosphate, although the above-mentioned ones may be used, phytic acid is preferably used, in particular.

As described above, the filled content may contain at least one selected from the group consisting of phospholipids, amino sugars, and organic acids, and the case in which phospholipids and organic acids are contained is particularly preferable.

Although the amount of these added is not particularly limited, an extremely small addition cannot demonstrate sufficient effects due to the addition, while an extremely large addition relatively decreases the amount of an active ingredient of pharmaceuticals or the like. Accordingly, it is preferable that the total amount of an addition with respect to the total amount of the filled content be 0.05 to 20% by mass, more preferably 0.1 to 10% by mass, and even more preferably 0.5 to 10% by mass.

Although the present invention can exhibit effects in spite of the constitution of the filled content, it is particularly effective when the filled content contains at least one selected from the group consisting of galenicals, unsaturated fatty acids, unsaturated fatty acid residue-containing oils and fats, minerals, mineral-containing yeasts, and vitamin C, because these significantly decrease the solubility of gelatin coating in the prior art.

The term "galenicals" refers to those used in raw materials for pharmaceuticals (including Chinese medicinal drugs and folk medicines), perfumery, or spices, without modification or after being simply processed or prepared by, for example, cutting, crushing, and drying a part or all of an animal, a plant, or a mineral without changing the nature thereof. In the capsule, the galenicals may be used in the form of powder, extract, essence, tincture, or the like.

Specific examples of galenicals include plants such as Mallotus bark, Gambir, Aloe, Epimedium herb, Fennel, Mume fruit, Lindera root, Bearberry leaf, Turmeric, Rose fruit, Acanthopanacis cortex, Corydails tuber, Rabdosiae herb, Milkvetch root, Scutellaria root, Solomonseal rhizome, Phellodendron bark, Japanese cherry bark, Coptis rhizome, Polygala root, Phocae testis et penis, SEA horse, Polygonum tuber, Zedoary, Puerariae radix, Valerianae radix, Guarana, Glycyrrhiza, Platycodon root, Immature orange, Apricot kernel, Barbary wolfberry fruit, Schizonepeta spike, Cinnamon bark, Cassia seed, Gentian, Geranium herb, Red ginseng, Magnolia bark, Oriental bezoar, Acanthopanacis cortex, Achyranthes root, Evodia fruit, Schisandra fruit, Bupleurum root, Asiasari root, Thyme, Sage, Smilax rhizome, Hawthorn fruit, Gardenia fruit, Cornus fruit, Zanthoxylum fruit, Zizyphus seed, Dioscorea rhizome, Rehmannia root, Civet, Peony root, Cnidium fruit, Plantago herb, Houttuynia herb, Amomum seed, ginger, Cardamon, Glossy privet fruit, Earthworm, Magnoliae flos, Senega, Cnidium rhizome, Peucedani radix, Swertia herb, Atractylodis lanceae rhizoma, Mori cortex, Perillae herba, Rhubarb, Zizyphi fructus, Clove, Gambir plant, Citrus unshiu peel, Capsicum, Japanese angelicae root, Tangshen, Peach kernel, Bitter orange peel, Ipecac, Dodder seed, Eucommia bark, Nandinae fructus, Nanbange, Cistanchis herb, garlic, Ophiopogonis tuber, Glehnia root, Pinellia tuber, Agkistrodon j aponicae, Atractylodis rhizoma, Poria sclerotium, Sinomenium stem, Malaytea scurfpea fruit, Moutan cortex, Hop, Ephedra herb, Actinidiae fructi galla, Muira puama, Saussureae radix, Coicis semen, Longan arillus, Gentianae scabrae radix, Scopolia rhizome, Cervi parvum cornu, Chrysanthemum flower, Oat leaflet, Safflower, Salacia, Honeysuckle stem, Ginseng (such as Panaxginseng, Panaxnotoginseng, or the like), Artemisia, green tea, herbs (such as ginkgo biloba, St John's wort, chamomile, Piper methysticum, blueberry, bilberry, Serenoa repens, Salacia oblonga, garcinia cambogia, rosemary, citrus, Vinca minor, echinacea, and the like), and the like, extracts, essences, or tinctures thereof;

powders obtained by drying and pulverizing without modification fungus (fruit body) such as Agaricus, Phellinus linteus, Ganoderma lucidum, Flammulina veluptipes, Schizophyllum commune, shiitake mushroom, Grifola frondosa, Chaga (Fuscoporia obliqua), mushroom, Lyophyllum decastes, Coriolus versicolor, Crepidotus mollis, bracket fungus, Cordyceps sinensis saccardo, Ganoderma lucidum, or the like, essences extracted from the fungus using hot water (which may include ethanol), essence powders, and the like;

extracts of animals, hydrolysates obtained by treating the extracts using acids, bases, or enzymes, those collected from the nests of animals (such as, for example, ox bile, chondroitin, glucosamine, collagen, propolis, and the like);

essences of fermented substances obtained by fermenting cereals, plants, marine products, or the like, using koji molds, red koji molds, lactic acid bacterias, acetic acid bacterias, bacillus natto, yeasts, or the like;

Chinese medicinal drugs composed of a combination of galenicals such as, for example, Kakkonto (Puerariae radix,

EP 1 790 336 A1

Zizyphi fructus, Peony root, ginger, Ephedra herb, Cinnamon bark, Glycyrrhiza), Tokishokuyakusan (Japanese angelicae root, Notopterygii rhizome, Peony root, Poria sclerotium, Atractylodis lanceae rhizoma, Alismatis rhizoma), Hachimijio (Rehmannia root, Dioscorea rhizome, Poria sclerotium, Cinnamon bark, Cornus fruit, Alismatis rhizoma, Moutan cortex, Aconiti tuber), Shoseiryuto (Ephedra herb, ginger, Cinnamon bark, Schisandra fruit, Peony root, Glycyrrhiza, Asiasari root, Pinellia tuber), Bakumondoutou (Ophiopogonis tuber, Oryzae fructus, Ginseng, Pinellia tuber, Zizyphi fructus, Glycyrrhiza), Kami-shoyosan (Japanese angelicae root, Atractylodis lanceae rhizoma, Bupleurum root, Gardenia fruit, ginger, Peony root, Poria sclerotium, Moutan cortex, Glycyrrhiza, Mentha herb), and the like.

The filled content may include at least one of these galenicals.

The present invention is particularly effective when the filled content includes rosemary, citrus, blueberry, bilberry, propolis, Panaxnotoginseng, Panaxginseng, Agaricus, Phellinus linteus, shiitake mushroom, Ganoderma lucidum, and essences thereof, because the effect of preventing the decrease in coating solubility is significant.

Although the "unsaturated fatty acid" is not particularly limited, the present invention is particularly effective when the filled content contains a long-chain unsaturated fatty acid having at least 14 carbon atoms, and more preferably 14 to 22 carbon atoms, because the effect of preventing the decrease in coating solubility is significant.

Examples of the long-chain unsaturated fatty acid having at least 14 carbon atoms include DHA (docosahexaenoic acid), EPA (eicosapentaenoic acid), linoleic acid, arachidonic acid, oleic acid, pinolenic acid, sciadonic acid, Jinoiperon acid, columbinic acid, conjugated linoleic acid, eleostearic acid, octadecenoic acid, octadecadienoic acid, docosenoic acid, ricinoleic acid, α-linolenic acid, γ-linolenic acid, behenic acid, and the like. The filled content may contain at least one of these unsaturated fatty acids. The present invention is particularly effective when the filled content contains DHA and/or EPA.

The unsaturated fatty acid may be formulated in the form of an isolated product of the unsaturated fatty acid or oil containing the same.

The phrase "unsaturated fatty acid residue-containing oils and fats" refers to oils and fats in which at least one of fatty acid residues composing the oils and fats is an unsaturated fatty acid residue.

Examples of the oils containing at least one of the unsaturated fatty acid residue-containing oils and fats include vegetable oils such as soybean oil, rape seed oil, rice bran oil, cotton seed oil, sesame oil, sunflower oil, mustard oil, safflower oil, corn oil, peanut oil, olive oil, palm oil, coconut oil, and the like, and animal oils such as fish oil, whale oil, beef tallow, lard, milk fat, and the like. The filled content may contain at least one of these oils.

The present invention is particularly effective when the filled content contains a fish oil, because many unsaturated fatty acids (DHA and EPA) described above are also contained in the unsaturated fatty acid residue-containing oils and fats, and the effect of preventing the decrease in coating solubility is significant.

The "mineral" is not particularly limited, and it refers to an inorganic substance that is useful from a nutritional standpoint. Examples thereof include calcium, phosphorus, iron, sodium, potassium, magnesium, zinc, selenium, copper, and the like. Among them, the present invention is particularly effective when calcium, phosphorus, iron, magnesium, zinc, selenium, or copper is contained, because use of polyvalent elements as a content filled in a conventional capsule significantly causes the insolubilization of the coating thereof, but the insolubilization can be inhibited in the capsule of the present invention. These may be used as inorganic or organic salts used for common foods.

The "mineral-containing yeast" is not particularly limited, and examples thereof include magnesium-containing yeasts, zinc-containing yeasts, selenium-containing yeasts, iron-containing yeasts, copper-containing yeasts, and the like. The mineral-containing yeast is one in which a mineral is incorporated in the yeast body.

The filled content may arbitrarily contain, in addition to the above-mentioned components, additives generally used for pharmaceuticals, food, or the like, such as excipients, bonding agents, disintegrators, stabilizers, dispersants, coloring agents, flavoring compounds, medium-chain fatty acid monoglycerides, medium-chain fatty acid triglycerides, polyethylene glycols, surfactants (glycerin fatty acid esters, or the like), antioxidants (vitamin E, astaxanthin, catechin, or the like), or the like, as needed.

Since the capsule of the present invention includes the capsule coating of the present invention, the decrease in solubility over time is significantly prevented, and particularly in the case of a soft capsule, a sufficient strength is given to the capsule.

Particularly, formulation of at least one selected from the group consisting of phospholipids, amino sugars, and organic acids in the filled content can further prevent the decrease in solubility, and is preferable.

[Examples]

In the following, although test examples with respect to the present invention will be mentioned, the present invention is not limited to these.

Effect of Inhibiting Decrease in Coating Solubility

"Test Example 1" (Examples 1-1, 1-2 and Comparative Examples 1-1 to 1-3)

(Preparation of Gelatin Coating)

100 g of gelatin and 45 g of glycerin were added to 100 g of purified water to make them absorb water and swell, followed by dissolving them at approximately 80˚C to prepare a gelatin solution. To this solution, each organic acid of which the kind and content are indicated in Table 1 (the concentration shown in the table represents the concentration (% by mass) with respect to the total amount of composition) was added, mixed, and then deaerated under reduced pressure to prepare a composition for capsule coating.
Each obtained composition was poured into a TLC plate, uniformly spread to a thickness of 1 mm, and then dried at 30˚C for 24 hours to obtain a film-like gelatin coating having a moisture content of approximately 9%. The coating obtained in each example was cut into a 1 cm x 1 cm piece, and used for the following evaluation.

(Evaluation)

15 ml of DHA (fish oil) was poured in a screw tube, into which two pieces of each gelatin coating piece prepared in the respective example were immersed, and stored at 50˚C. After one or two day(s), the gelatin coating pieces were taken out, and the content adhered to the pieces was removed, followed by putting the pieces in 200 ml of hot water at 60˚C and stirring them with a stirrer bar for 2 minutes. The pieces were visually observed in the state of repose, and evaluated in accordance with the following criteria.
Criteria for Evaluation
(-): The gelatin coating pieces were completely dissolved, and undissolved pieces thereof were not recognized.
(±): Undissolved pieces were slightly recognized.
(+): Undissolved pieces were recognized in small amounts.
(++): Undissolved pieces were recognized in medium amounts.
(+++): Undissolved pieces were recognized in large amounts.
(++++): The gelatin coating pieces were not dissolved at all, and completely insolubilized.

(Results)

Results are shown in Table 1.
In Comparative Examples 1-1 to 1-3, the gelatin coating was completely insolubilized after two days, while in Examples 1-1 and 1-2 in which inositol hexaphosphate (phytic acid) was added, the gelatin coating was completely dissolved even after two days. Accordingly, it was apparent that addition of inositol hexaphosphate significantly prevented the decrease in solubility of the gelatin coating over time.
Table 1

| Effect of Inhibiting Decrease in Solubility of Gelatin Coating in DHA | | | | | |
|---|---|---|---|---|---|
| | Example 1-1 | Example 1-2 | Comparative Example 1-1 | Comparative Example 1-2 | Comparative Example 1-3 |
| Organic Acid (% by mass) | Inositol Hexaphosphate (2%) | Inositol Hexaphosphate (4%) | Additive-Free | Citric Acid (2%) | Citric Acid (4%) |
| First Day | - | - | ++ | + | + |
| Second Day | - | - | ++++ | ++++ | ++++ |

"Test Example 2" (Examples 2-1 to 2-13)

(Preparation of Soft Capsule)

Each composition for the capsule coating composed of 45% by mass of gelatin, 18% by mass of glycerin, inositol phosphate of the kind in an amount as indicated in Table 2 (the concentration shown in the table represents the concentration (% by mass) with respect to the total amount of respective composition), and water (the remnant) was produced in the same way as that of Test Example 1, followed by deaerating and being kept for 10 hours to use for producing capsules.

Each component of which the kind and content are indicated in the same table was filled using a rotatory type soft capsule filler (Oval Type 5). After filling, each capsule coating was dried at 27°C under 50% or lower humidity till the moisture content in the capsule coating became 8% and so a soft capsule was prepared.

Table 2

| Example | Inositol Phosphate Added to Coating (% by mass) | Filled Content (Amount) |
|---------|--------------------------------------------------|-------------------------|
| 2-1 | Inositol Hexaphosphate (1 %) | DHA (Fish Oil) (300 mg) |
| 2-2 | Inositol Hexaphosphate (2%) | DHA (Fish Oil) (300 mg) |
| 2-3 | Inositol Hexaphosphate (5%) | DHA (Fish Oil) (300 mg) |
| 2-4 | Inositol Hexaphosphate (8%) | DHA (Fish Oil) (300 mg) |
| 2-5 | Inositol Hexaphosphate (10%) | DHA (Fish Oil) (300 mg) |
| 2-6 | Inositol Hexaphosphate (4%) | Ganoderma Lucidum (130 mg) Wheat Germ Oil (150 mg) |
| 2-7 | Inositol Hexaphosphate (4%) | Panaxnotoginseng (100 mg) Propolis (150mg) |
| 2-8 | Inositol Hexaphosphate (2%) | DHA (Fish Oil 1)(150mg) EPA (Fish Oil) (80 mg) |
| 2-9 | Inositol Hexaphosphate (2%) | Blueberry (120 mg) DHA (Fish Oil) (150 mg) |
| 2-10 | Inositol Pentaphosphate (2%) | DHA (Fish Oil) (300 mg) |
| 2-11 | Inositol Triphosphate (2%) | DHA (Fish Oil) (300 mg) |
| 2-12 | Inositol Hexaphosphate (4%) | Vitamin C (100 mg) Wheat Germ Oil (150 mg) |
| 2-13 | Inositol Hexaphosphate (4%) | Hemoferrum (100 mg) Zinc-containing Yeast (50 mg) Wheat Germ Oil (150 mg) |

Effect of Giving Strength to Capsule

"Test Example 3" (Example 3 and Comparative Example 3)

(Preparation of Gelatin Coating and Soft Capsule)

A gelatin solution (A) composed of 45% by mass of gelatin, 18% by mass of glycerin, 1% by mass of inositol hexaphosphate (phytic acid), and 36% by mass of water, and a gelatin solution (B) composed of 45% by mass of gelatin, 18% by mass of glycerin, and 37% by mass of water were each prepared, dissolved at 80°C, deaerated, and then kept for about 10 hours. Each gelatin coating with a thickness of 0.90 mm was formed from the gelatin solution (A) or (B) using a rotatory type soft capsule filler (Oval Type 5), into which 300 mg of DHA (fish oil) was filled to prepare soft capsules. The capsules were evaluated as described below.

(Evaluation)

Immediately after starting to fill each, filled capsules No. 1, No. 4, and No. 7 (front, middle, back) were sampled from capsule filler metal molds No. 1 to No. 7, and each content thereof was removed therefrom, followed by cutting in round slices to measure the thickness of the thinnest portion of the joined portion thereof using a microscope with a scale for comparison. Moreover, after operating the filler for 1 hour using the respective gelatin solution, filled capsules were sampled in the same way as described above, and the thickness of the thinnest portion of the joined portion thereof was measured for evaluation. Next, the adhesion ratio was calculated from the thickness of the joined portion for evaluation.

(Results)

Results are shown in Table 3 and Table 4.

In comparison with Comparative Example 3 in which the capsules were produced using the gelatin solution (B), Example 3 in which the capsules were produced using the gelatin solution (A) containing inositol hexaphosphate (phytic acid) demonstrated a high adhesion ratio and it was confirmed that the strength of the capsules increased.

Table 3

| Thickness of Joined portion | | | | | | (Unit: mm) |
|---|---|---|---|---|---|---|
| | | | Metal mold No.1 (Front row) | Metal mold No.4 (Middle row) | Metal mold No. 7 (Back row) | Average (Av) |
| Example 3 | Gelatin solution (A) | Immediately after filling | 0.45 | 0.46 | 0.43 | 0.45 |
| | | After 1 hour | 0.45 | 0.47 | 0.42 | 0.45 |
| Comparative Example 3 | Gelatin solution (B) | Immediately alter filling | 0.32 | 0.36 | 0.32 | 0.33 |
| | | After 1 hour | 0.33 | 0.35 | 0.34 | 0.34 |

Table 4

| Adhesion Ratio | | | | (Unit: %) |
|---|---|---|---|---|
| | | Immediately after filling | After 1 hour | Average (Av) |
| Example 3 | Gelatin solution (A) | 50.0 | 50.0 | 50 |
| Comparative Example 3 | Gelatin solution (B) | 36.7 | 37.8 | 37 |

※ Adhesion Ratio (%) = (Average Adhesion Thickness (mm) / 0.90 mm) × 100

INDUSTRIAL APPLICABILITY

The present invention can be preferably applied for a capsule used in the field of pharmaceuticals, quasi-drugs, health foods, general foods, cosmetics, or the like.

**Claims**

1. A composition for a capsule coating **characterized by** comprising a gelatin (A) and an inositol phosphate (B) represented by a general formula: $C_6H_{12-n} \cdot (H_2PO_4)_n$ (wherein n represents an integer from 1 to 6).

2. A capsule coating **characterized by** comprising a gelatin (A) and an inositol phosphate (B) represented by a general formula: $C_6H_{12-n} \cdot (H_2PO_4)_n$ (wherein n represents an integer from 1 to 6).

3. A capsule coating according to claim 2, **characterized in that** the component (B) comprises an inositol hexaphosphate.

4. A capsule **characterized by** comprising the capsule coating of claim 2 or 3.

5. A capsule according to claim 4, **characterized in that** a filled content thereof comprises at least one selected from the group consisting of galenicals, unsaturated fatty acids, unsaturated fatty acid residue-containing oils and fats, minerals, mineral-containing yeasts, and a vitamin C.

6. A capsule according to claim 4 or 5, **characterized in that** a filled content thereof comprises at least one selected from the group consisting of phospholipids, amino sugars, and organic acids.

7. A capsule according to any one of claims 4 to 6, **characterized by** being a soft capsule.

8. A production method of the capsule of claim 7, **characterized by** comprising a production step of a composition for a capsule coating containing at least a gelatin (A) and an inositol phosphate (B) represented by a general formula: $C_6H_{12-n} \cdot (H_2PO_4)_n$ (wherein n represents an integer from 1 to 6) and an encapsulation step in which the capsule coating is produced from the composition for the capsule coating and a content to be filled in the capsule is interposed between the capsule coating followed by conducting pressure bonding.

9. A production method according to claim 8, **characterized by** further comprising a step of preparing the content to be filled containing at least one selected from the group consisting of phospholipids, amino sugars, and organic acids.

## INTERNATIONAL SEARCH REPORT

| International application No. |
| --- |
| PCT/JP2005/013916 |

A.  CLASSIFICATION OF SUBJECT MATTER
*A61K9/64* (2006.01), *A61K9/56* (2006.01), *A61K31/19* (2006.01),
*A61K31/661* (2006.01), *A61K31/7008* (2006.01), *A61P3/00* (2006.01),
*A23L1/00* (2006.01)

According to International Patent Classification (IPC) or to both national classification and IPC

B.  FIELDS SEARCHED

Minimum documentation searched (classification system followed by classification symbols)
*A61K9/64* (2006.01), *A61K9/56* (2006.01), *A61K31/19* (2006.01),
*A61K31/661* (2006.01), *A61K31/7008* (2006.01), *A61P3/00* (2006.01),
*A23L1/00* (2006.01)

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN), JSTPlus(JOIS)

C.  DOCUMENTS CONSIDERED TO BE RELEVANT

| Category* | Citation of document, with indication, where appropriate, of the relevant passages | Relevant to claim No. |
| --- | --- | --- |
| A | JP 9-202727 A  (KAO CORP), 05 August, 1997 (05.08.97), Full text; particularly, Claims 1, 4 (Family: none) | 1-9 |
| A | JP 2002-524151 A  (ALZA CORP), 06 August, 2002 (06.08.02), Full text; particularly, Claim 11; Par. No. [0031] & WO 2000/013674 A1 | 1-9 |
| A | JP 2003-169632 A  (SAPURI KABUSHIKI KAISHA), 17 June, 2003 (17.06.03), Full text (Family: none) | 1-9 |

☐ Further documents are listed in the continuation of Box C.   ☐ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 19 October, 2005 (19.10.05) | 01 November, 2005 (01.11.05) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**EP 1 790 336 A1**

**REFERENCES CITED IN THE DESCRIPTION**

**Patent documents cited in the description**

- JP 2004223745 A
- JP 2005129968 A
- JP 57030088 B
- JP 59039834 A
- JP 5065222 A